# FASCICULE DE BREVET EUROPEEN

(11) **EP 2 864 273 B1**
(45) Date de publication et mention de la délivrance du brevet: **27.07.2022**
(21) Numéro de dépôt: 12733514.9
(22) Date de dépôt: 21.06.2012
(51) Int. Cl.: C04B 35/645, G01T 1/24, H01L 31/0296, H01L 31/032, H01L 31/08, C04B 35/01, C04B 35/515

(54) **PROCÉDÉ DE FABRICATION D'UNE PASTILLE POUR DÉTECTEUR À CONVERSION DIRECTE DE RAYONS X, DÉTECTEUR À CONVERSION DIRECTE DE RAYONS X ET APPAREIL DE RADIOLOGIE DENTAIRE UTILISANT UN TEL DÉTECTEUR**
VERFAHREN ZUR HERSTELLUNG VON PELLETS FÜR EINEN DIREKTWANDLUNGSDETEKTOR FÜR RÖNTGENSTRAHLEN, DIREKTWANDLUNGSDETEKTOR FÜR RÖNTGENSTRAHLEN UND ZAHNRÖNTGENVORRICHTUNG MIT SOLCH EINEM DETEKTOR
METHOD FOR PRODUCTION OF A PELLET FOR A DIRECT-CONVERSION DETECTOR OF X-RAYS, DIRECT-CONVERSION DETECTOR OF X-RAYS AND DENTAL RADIOLOGY APPARATUS USING SUCH A DETECTOR

(43) Date de publication de la demande: 29.04.2015
(73) Titulaire: Trophy, 77435 Marne La Vallée Cedex 2 (FR)
(72) Inventeur: BARBOUX, Philippe, F-94240 L'Hay les Roses (FR); BIAVA, Dominique, F-92140 Clamart (FR); BINET, Laurent, F-94400 Vitry sur Seine (FR); BOTHOREL, Sylvie, F-77435 Marne La Vallée Cedex 2 (FR); GOURIER, Didier, F-75013 Paris (FR); INGLESE, Jean-Marc, F-77600 Bussy St Georges (FR); PONPON, Jean-Pierre, F-67201 Echbolsheim (FR); RAULT, Mathieu, F-75005 Paris (FR)
(74) Mandataire: Wimmer, Hubert
(86) Numéro de dépôt international: PCT/FR2012/051402
(87) Numéro de publication internationale: WO 2013/190187

(56) Documents cités:
- US-A- 5 892 227
- SCHIEBER M ET AL: "Theoretical and experimental sensitivity to X-rays of single and polycrystalline HgI2 compared with different single-crystal detectors", NUCLEAR INSTRUMENTS & METHODS IN PHYSICS RESEARCH. SECTION A: ACCELERATORS, SPECTROMETERS, DETECTORS, AND ASSOCIATED EQUIPMENT, ELSEVIER BV * NORTH-HOLLAND, NETHERLANDS, vol. 458, no. 1-2, 1 février 2001 (2001-02-01), pages 41-46, XP027324597, ISSN: 0168-9002 [extrait le 2001-02-01]
- TURCHETTA R ET AL: "Imaging with polycrystalline mercuric iodide detectors using VLSI readout", NUCLEAR INSTRUMENTS & METHODS IN PHYSICS RESEARCH. SECTION A: ACCELERATORS, SPECTROMETERS, DETECTORS, AND ASSOCIATED EQUIPMENT, ELSEVIER BV * NORTH-HOLLAND, NETHERLANDS, vol. 428, no. 1, 1 juin 1999 (1999-06-01), pages 88-94, XP004169921, ISSN: 0168-9002, DOI: 10.1016/S0168-9002(98)01584-8

## Description

### DOMAINE TECHNIQUE DE L'INVENTION

La présente invention concerne un procédé de fabrication d'une pastille d'un matériau semi-conducteur pour un détecteur à conversion directe de rayons X. Elle concerne aussi un détecteur à conversion directe de rayons X utilisant une telle pastille et un appareil de radiologie dentaire utilisant au moins un tel détecteur.

### ETAT DE LA TECHNIQUE

Dans l'état de la technique, on a déjà proposé des appareils de radiologie, notamment dentaire, qui permettent d'obtenir des images en deux ou trois dimensions (2D ou 3D) de l'intérieur de la bouche. Une source de rayons X est disposée en relation avec un détecteur de rayons X de sorte que l'image par transmission à travers la tête du patient soit formée sur un support donné en relation avec le détecteur de rayons X.

Une première exigence particulière à la radiologie dentaire est que la quantité de rayons X nécessaire pour obtenir une image doit être aussi faible que possible. En effet, à forte dose, les rayons X peuvent présenter un effet néfaste sur la santé. Non seulement, les doses de rayons X reçues par le patient doivent être strictement réduites, mais celles reçues de manière parasite par l'opérateur doivent aussi être réduites selon des normes internationales extrêmement sévères.

Parmi tous les détecteurs connus de rayons X, est apparu un type particulier de détecteurs de rayons X dénommé détecteur à conversion directe de rayons X.

Alors qu'un détecteur à conversion indirecte possède un scintillateur qui a pour rôle de transformer un photon X reçu en un photon lumineux, qui sera ensuite converti en charges électriques à l'aide d'un convertisseur photonique classique, le détecteur à conversion directe transforme directement un photon X reçu en charges électriques. Ces charges sont ensuite collectées en appliquant un champ électrique.

A cet effet, un détecteur à conversion directe comporte une pastille, dite pastille semi-conductrice qui produit un courant électrique dû à la migration sous l'effet d'un champ électrique des charges créées par l'absorption d'un photon X.

Un objet de la présente invention est de fabriquer et de mettre en forme le matériau semi-conducteur sous la forme d'une pastille semi-conductrice céramique pour réaliser la détection directe d'un flux de photons X en un courant électrique dans le but de réaliser une image 2D ou 3D d'une scène éclairée par une source de rayons X.

En radiologie dentaire, on note par ailleurs que plusieurs techniques exigent des caractéristiques particulières des détecteurs à conversion directe de rayons X. En effet, la production de radiographies panoramiques révélant entièrement les deux arcs dentaires et la production d'images, notamment par technique orthopantomographique, exigent de pouvoir répéter rapidement plusieurs acquisitions d'images, et que le détecteur à conversion directe de rayons X doit détecter rapidement.

C'est un autre objet de l'invention de fabriquer une nouvelle pastille pour réaliser la conversion directe de plusieurs flux rapidement successifs de photons X en au moins un courant électrique dans le but de réaliser une séquence d'images 2D d'une scène éclairée par une source de rayons X

Les détecteurs à conversion directe de rayons X produits selon l'état de la technique souffrent en effet de certaines limitations, liées au matériau semi-conducteur. De plus, les méthodes actuelles d'obtention du matériau semi-conducteur présentent certains inconvénients.

Idéalement, le matériau semi-conducteur doit être bon marché et facile à réaliser.

La dangerosité des étapes de préparation de certains matériaux semi-conducteurs est un frein à leur développement, en particulier pour leur usage dans des détecteurs de rayons X.

Le matériau doit permettre de réaliser des capteurs de taille suffisamment grande.

Le matériau doit présenter une faible porosité, afin de ne pas diminuer ses propriétés d'absorption des rayons X. De plus, la porosité constitue une barrière pour le déplacement des charges électriques et peut être une cause de capture de ces charges et détériore et donc les propriétés de transport électrique du matériau.

L'état de surface des faces opposées de la pastille réalisée avec un tel matériau doit être très uniforme pour favoriser la pose d'électrodes sur ces deux faces et pour garantir une rugosité suffisamment faible pour assurer un champ électrique homogène.

Le matériau doit avoir une composition aussi proche que possible de la stœchiométrie de Hg12, reproductible et constante dans tout son volume. En effet, un écart par rapport à la stœchiométrie peut générer une phase parasite qui perturbe les lignes de champ électrique avec des conséquences sur la netteté de l'image et elle peut créer des défauts qui peuvent piéger les charges électriques et ainsi dégrader la sensibilité du détecteur.

Dans la suite de la demande, on utilisera le plus souvent la sensibilité du détecteur comme le rapport entre la grandeur de sortie (quantité d'électricité en coulomb) par la grandeur d'entrée (fluence de l'énergie ionisante des photons X, soit la dose d'entrée par unité de surface en gray x cm2). La sensibilité du détecteur pourra être exprimée en sensibilité relative sur cette base.

Certains matériaux semi-conducteurs sont anisotropes, tel l'iodure de mercure HgI2. Ces matériaux anisotropes présentent une ou plusieurs caractéristiques qui dépendent de l'orientation du matériau. Dans le cas de l'iodure de mercure, la résistance au déplacement des charges électriques est plus faible selon l'axe cristallographique « *c* ». Il est alors important que cet axe C soit orienté selon la direction de conduction électrique depuis la face externe du matériau jusqu'au capteur lorsque le matériau semi-conducteur anisotrope est intégré dans le détecteur.

Les monocristaux de type CdTe ou HgI₂ sont constitués d'un unique grain et présentent donc une porosité nulle. Un tel matériau est néanmoins fastidieux et délicat à préparer. La réalisation de pastilles de faible épaisseur, avec des surfaces de contact nettes, à partir du barreau monocristallin n'est pas économiquement faisable. La croissance de couches de HgI₂ peut être réalisée sur un substrat semi-conducteur de type CMOS par dépôt sous vide en phase vapeur, (méthode PVD). L'iodure de mercure est porté en phase vapeur à 90°C et se condense sur le substrat froid à 70°C. Les avantages d'une telle croissance sont que le matériau semi-conducteur est déposé directement sur le substrat (circuit électronique de lecture), et que l'axe C, orienté selon la direction de croissance des cristaux, est orthogonal à la surface du substrat, et cet axe est par conséquent orienté selon la direction de conduction facile des charges électriques dans le mode de fonctionnement du détecteur ainsi obtenu.

Néanmoins, cette méthode présente de nombreux inconvénients. Le mode de préparation présente une certaine dangerosité pour la santé puisqu'il nécessite la manipulation de mercure ou de HgI₂ à l'état gazeux. Elle est techniquement difficile à réaliser puisqu'elle exige une fabrication sous un vide rigoureusement contrôlé et des températures de HgI₂ et du substrat très précisément définies. La composition et la stoechiométrie sont très dépendantes des températures du HgI₂ en phase gazeuse, de la température du substrat et de la vitesse de dépôt. Enfin la planéité du substrat n'étant pas parfaite par rapport à la taille de la maille du cristal, le début de la croissance peut être perturbé à certains endroits et empêcher la conduction à l'interface matériau semi-conducteur /électrode.

Une deuxième méthode de l'état de la technique permettant d'obtenir des monocristaux d'un matériau semi-conducteur tel que le HgI₂ consiste à dissoudre de la poudre de HgI₂ dans un solvant à chaud. On refroidit ensuite le solvant et on le laisse s'évaporer avant de récolter le monocristal. Cette méthode est plus facile à mettre en œuvre que la précédente mais non reproductible. Elle ne permet pas de contrôler la taille et la forme des monocristaux. De plus, les monocristaux ainsi produits ont une pureté et une composition mal contrôlées, un mauvais état de surface et des dimensions de l'ordre du millimètre incompatibles avec des détecteurs de grande surface.

Une troisième méthode de l'état de la technique consiste à mélanger de la poudre d'un matériau semi-conducteur tel que HgI₂ dans un liant polymère (Méthode dite "PIB" - « Particule In Binder »). Le polymère a des propriétés adhésives qui lient entre eux en un matériau rigide les grains de HgI₂. Les avantages d'une telle méthode sont la facilité de préparation et la possibilité de coller facilement le matériau ainsi obtenu sur le capteur CMOS. Les inventeurs ont réalisé une analyse des inconvénients du polycristal ainsi obtenu. Il subsiste notamment une forte porosité entre les grains du matériau semi-conducteur et une dispersion de l'orientation de l'axe C au sein du matériau dans le cas d'un matériau semi-conducteur anisotrope.

Une quatrième méthode de l'état de la technique consiste à préparer le matériau semi-conducteur par frittage. Le frittage est un procédé de fabrication de céramique consistant à faire croître les grains et à les souder entre eux avec un apport d'énergie en chauffant une poudre, en général préalablement compactée par pressage, sans la mener jusqu'à la fusion. Sous l'effet de la chaleur, les grains se soudent entre eux par inter-diffusion de matière, ce qui permet la cohésion de la pièce. Néanmoins, cette méthode ne permet pas d'obtenir un matériau aux propriétés de transport électrique satisfaisantes. En effet, le matériau ainsi obtenu serait très poreux et sans orientation préférentielle de l'axe C.

Dans la demande internationale WO-A-00/68999 publiée le 16 novembre 2000, il est décrit un tel état de la technique dans lequel on réalise par frittage une céramique à base de CdTe. Il y est proposé d'effectuer une étape préalable de mise en forme de la pastille avant le frittage à l'aide d'une matrice de compaction. Cette compaction de la poudre initiale est effectuée avant de réaliser le chauffage, et donc le frittage, et elle constitue une simple mise en forme de la pastille. Un tel traitement sur une telle sélection du CdTe ne permet pas d'obtenir les résultats visés par l'invention.

Dans le brevet US-A-5.892.227 (publié aussi sous forme de la demande internationale numéro WO-A-96/10194 le 4 avril 1996) parmi deux autres techniques de dépôt en suspension et de dépôt en phase vapeur, il est aussi décrit une technique de frittage appliquée à une poudre d'un produit ou d'un mélange de produits semi-conducteurs. La mise en température dans un four est accompagnée par une mise en pression à l'aide de pistons appliqués sur la pastille.

Cependant, la méthode de frittage n'est citée dans ce document que parmi plusieurs méthodes qui y sont toutes tenues pour avantageuses, alors qu'on a pu montrer qu'elles ne l'étaient pas.

Lors d'essais, il a en effet été possible de déterminer plusieurs défauts de cet état de la technique. Le premier défaut est que le matériau semi-conducteur est un polycristal en poudre tel que du iodure de mercure HgI₂ et qu'il doit être préalablement traité pour atteindre un degré très élevé de pureté. Il en découle une source de coûts supplémentaires liés au fait que la poudre utilisée au début du procédé de fabrication de la pastille doit d'abord être soumise à un processus de purification.

Un deuxième défaut de cet état de la technique est que l'un des matériaux proposés (en l'espèce l'or) pour les électrodes - qui permettent de récupérer les charges électriques produites lors de la conversion des photons X dans le matériau semi-conducteur de la pastille ainsi frittée - est un métal qui réagit chimiquement lors du frittage à chaud avec le matériau semi-conducteur de la poudre, en l'espèce HgI2. Il en résulte une dégradation et parfois même une destruction locale de l'électrode ou des électrodes.

La présente invention vise à résoudre ces problèmes et défauts de l'état de la technique.

### BREF RESUME DE L'INVENTION

Conformément à la présente invention, un procédé de fabrication d'une pastille semi-conductrice, une pastille semi-conductrice, un détecteur et un appareil de radiologie tels qu'énoncés dans les revendications 1, 7, 8 et 10, respectivement, sont fournis. D'autres modes de réalisation de l'invention sont notamment divulgués dans les revendications dépendantes. A cet effet, l'invention propose un procédé de fabrication d'une pastille semi-conductrice pour un détecteur à conversion directe de rayons X, le procédé comportant une étape de mise en charge sous pression d'une poudre d'un matériau polycristallin semi-conducteur à base de PbI₂, de HgI₂ et/ou de PbO, et une étape de chauffage pendant une durée déterminée. Selon l'invention, le taux pureté de la poudre du matériau polycristallin semi-conducteur est supérieure ou égale à 99% et inférieure ou égale à 99.8%.

Selon d'autres caractéristiques du procédé :
- l'étape de mise en charge consiste à appliquer une force de compression axiale de la poudre du matériau polycristallin semi-conducteur d'une valeur assurant une orientation axiale C des grains du matériau polycristallin semi-conducteur selon la direction d'application C de ladite force de compression axiale ;
- la valeur de ladite force de compression axiale est comprise entre 100MPa et 1.000 MPa, et en ce que la durée de mise sous charge est égale à au moins une heure ;
- la température de chauffage est comprise entre 70°C et 200°C, et la durée de chauffage est égale à au moins une heure ;
- l'étape de mise en charge est mise en oeuvre dès le début de l'étape de chauffage ;
- l'étape de mise en charge est mise en oeuvre durant toute l'étape de chauffage.

L'invention propose aussi un détecteur à conversion directe de rayons X qui comporte une pastille semi-conductrice fabriquée selon l'invention.

Selon d'autres caractéristiques d'un tel détecteur :
- il est associé à un circuit intégré semi-conducteur, et il comporte une première électrode continue en contact avec une face d'entrée de la pastille, et une seconde électrode constituée par une pluralité de pavés conducteurs en contact avec la face opposée de la pastille, de façon à fournir un arrangement unidimensionnel ou bidimensionnel de pixels, lesdites première et seconde électrodes étant connectées électriquement avec le circuit intégré semi-conducteur associé sur une face duquel la pastille semi-conductrice a été déposée, le circuit intégré semi-conducteur étant arrangé de sorte qu'il produise une pluralité de signaux électriques représentatifs de l'intensité des rayons X reçus dans les divers pixels de ladite pastille semi-conductrice.

L'invention propose encore un appareil de radiologie, notamment de radiologie dentaire, utilisant au moins un détecteur à conversion directe de rayons X selon l'invention.

Un tel appareil :
- comporte au moins un détecteur à conversion directe de rayons X selon l'invention, et il comporte de plus au moins une source de rayons X commandée et un circuit de commande pour exécuter au moins un tir de rayons X en direction dudit au moins un détecteur à conversion directe de rayons X et pour en déduire par visualisation, impression et/ou enregistrement au moins une représentation graphique sur la base de la pluralité de signaux électriques produits par ledit au moins un détecteur à conversion directe de rayons X.
- est un appareil de radiologie dentaire intraoral ou extraoral.

### BREVE DESCRIPTION DES FIGURES

D'autres caractéristiques et avantages de la présente invention seront mieux compris à l'aide de la description et des dessins annexés dans lesquels :
- la figure 1 représente schématiquement un dispositif au moyen duquel on réalise certaines étapes du procédé selon l'invention ;
- les figures 2a et 2b sont des schémas expliquant l'effet résultant de la compression axiale ;
- la figure 3 représente schématiquement un détecteur à conversion directe de rayons X utilisant une pastille semi-conductrice fabriquée selon l'invention ; et
- la figure 4 est un schéma d'un mode particulier de réalisation d'un appareil de radiologie dentaire utilisant un détecteur à conversion directe de rayons X selon l'invention.

Selon l'invention, le procédé de fabrication utilise une poudre d'un matériau semi-conducteur.

Selon la littérature scientifique, les matériaux à base de PbI₂, de HgI₂ et/ou de PbO sont potentiellement les plus efficaces. Dans un mode préféré de réalisation, le matériau choisi est Hg12. De plus, lorsque que le matériau possède une mobilité électrique anisotrope, ce qui est le cas des matériaux cités précédemment, il est préférable qu'il soit mis en forme de telle sorte que le courant électrique s'écoule dans la direction de plus grande mobilité.

Dans l'état de la technique, ainsi qu'il a été décrit ci-dessus, les poudres utilisées pour réaliser par frittage une pastille de détection devaient subir une série d'étapes de purification pour utiliser un matériau semi-conducteur le plus pur possible.

Au contraire et de manière surprenante, les inventeurs ont observé que la présence d'un taux minimal d'impuretés permettait d'obtenir un rendement de conversion électrique des photons X de valeur bien plus élevée.

Les inventeurs constatent que, dans le cas d'une pastille de HgI₂ fabriquée à partir d'une poudre moins pure, on obtient de meilleurs résultats en termes de sensibilité de détection.

Les impuretés désirées et admises selon l'invention sont particulièrement les résidus métalliques contenues dans la poudre initiale ou provenant notamment des outils utilisés pour réduire en poudre et pour mesurer ou calibrer le diamètre moyen des particules de la poudre et du procédé de fabrication de l'industriel producteur de poudres.

Préférentiellement, la poudre de matériau semi-conducteur à utiliser pour réaliser le frittage de la pastille est d'une pureté de 99,0%, représentant un taux d'impuretés, notamment métalliques, inférieur à 1,0%.

Une telle poudre "non purifiée" est avantageusement disponible dans le commerce, en permettant ainsi de supprimer tous les coûts inhérents à une série d'étapes de purification. Particulièrement, les fabricants de poudres présentent commercialement leurs gradations de pureté de produits par décades. Les essais menés par les inventeurs ont montré une amélioration des résultats des poudres frittées en conversion directe de photons X à partir de la décade à 0,2% d'impuretés. Ceci représente un gain de plus de deux mille fois l'impureté exigée dans l'état de la technique notamment dans le document précité US-A-5.892.227.

Les inventeurs se sont alors rendu compte que l'application d'une charge de compression axiale à un tel matériau comportant un taux minimal déterminé d'impuretés favorisait aussi une amélioration du rendement de conversion électrique par la réorientation des grains le long d'un axe cristallographique C commun aux grains composant la poudre initiale. Cette caractéristique se combine avec la caractéristique de taux d'impuretés minimal dans un mode de réalisation préféré.

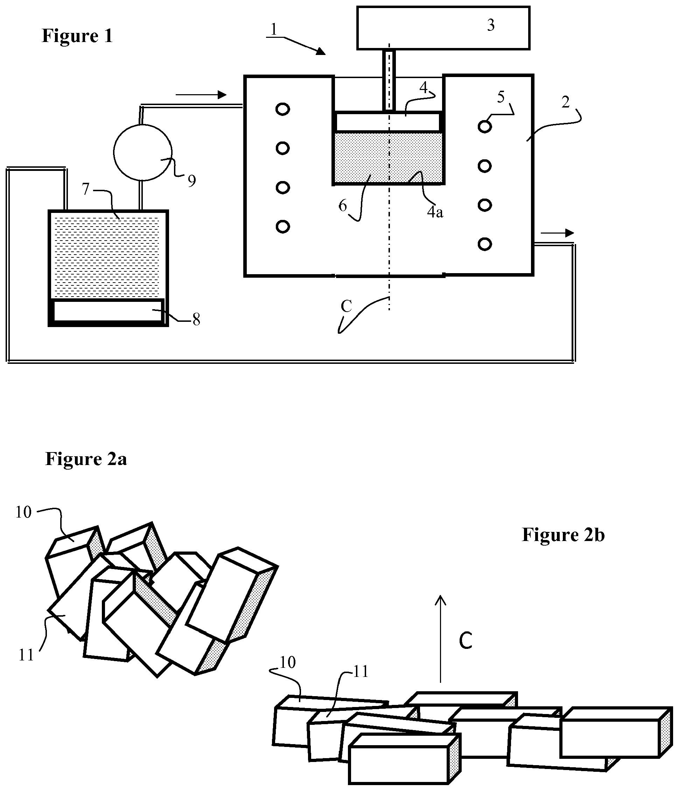

Sur la figure 1, on a représenté un dispositif permettant de mettre en œuvre le procédé de fabrication.

Un four 1 est constitué par un corps 2 conducteur de la chaleur, qui présente une ouverture centrale 6 limitée par une paroi inférieure 4a et par un piston de compression axiale 4 disposé au-dessus de la poudre de matériau semi-conducteur à fritter.

Un moule dont l'empreinte présente la forme que doit prendre la pastille de matériau semi-conducteur est disposé dans l'ouverture centrale ou logement 6 du four 1.

Un tel moule permet par exemple la production d'une pastille à au moins deux faces opposées parallèles et de contour circulaire ou rectangulaire.

Le corps 2 conducteur de chaleur est percé de canaux 5 de circulation d'une huile chauffée en provenance d'une réserve 7. L'huile de la réserve est chauffée par une résistance électrique 8 connectée par un circuit de contrôle de la durée et/ou de la température de frittage (non représenté).

L'huile chauffée est aspirée par une pompe électrique 9, commandée par le circuit de contrôle de la durée et/ou de la température de frittage (non représenté). L'huile chaude entre dans les canaux 5 du corps 2, ces canaux étant connectés en une forme hélicoïdale et le flux d'huile de chauffage est ensuite renvoyé vers la réserve 7.

Préférentiellement, le piston supérieur 4 est mis en action par un mécanisme 3 qui régule la valeur, et la durée d'application, de la force de compression axiale appliquée à la poudre, puis à la pastille semi-conductrice logée dans la chambre 6 du four 1.

Cette mise en action de la compression axiale est préférentiellement réalisée dés le début de la mise en chauffe, c'est à dire de la mise en température de frittage de la poudre puis de la pastille en fabrication.

Dans un autre mode de réalisation, on a aussi obtenu des résultats intéressants en exécutant une compression axiale à froid, donc avant d'activer le chauffage de la fabrication par frittage de la pastille de matériau semi-conducteur. Particulièrement, on a obtenu de bons résultats en exécutant un frittage sur bogue après une compression axiale à froid préalable allant jusqu'à une heure.

Sur la figure 2a, on a représenté schématiquement les grains dans une pastille de matériau semi-conducteur. Ces grains - représentés par exemple par les grains 10, 11 - ont une forme parallélépipédique. Cette forme ne préjuge pas de la forme réelle des grains dans la pastille, mais elle a pour but de mettre en évidence l'anisotropie de leurs propriétés électriques, dans le cas d'un matériau anisotrope. La direction C de plus grande mobilité électrique correspond à la dimension la plus petite du parallélépipède.

Les grains sont représentés sur la figure 2a orientés dans n'importe quelle direction. Il en résulte que si des charges électriques circulent à travers le matériau, celles-ci traverseront des grains selon leur direction de plus grande mobilité électrique, puis traverseront d'autres grains selon une direction de plus faible mobilité électrique. Il en résultera que la mobilité électrique globale du matériau ne sera pas maximale.

Sur la figure 2b, par la mise en oeuvre d'une compression axiale dans une direction C donnée, les faces des grains 10, 11 de la particule dans la poudre de matériau semi-conducteur ont tendance à quasiment s'aligner de telle sorte que la direction C de plus grande mobilité électrique soit parallèle à l'axe de compression axiale.

La mobilité électrique globale de la céramique le long de cette direction est donc augmentée. Il en résulte une amélioration de la circulation des charges électriques le long de cette direction, et par suite une amélioration du rendement de conversion électrique des photons X.

La compression ou mise sous charge, ou mise en charge, de la poudre à fritter a déjà été pratiquée dans l'état de la technique. Mais on note particulièrement que l'effet recherché était alors une simple compaction de la poudre, pour lui conférer une meilleure tenue mécanique avant que la pastille ainsi "pré-compactée" soit placée dans le four de frittage. En particulier, la compaction se traduit seulement par une réduction du volume de poudre, les vides entre particules de poudre étant réduits par cette mise en pression.

Selon l'invention, le fait de la compression axiale en même temps que le chauffage dépasse ce simple état de réduction de volume pour favoriser la croissance des grains ayant l'orientation de l'axe C parallèle à l'axe de compression au détriment des autres grains et aboutir ainsi à une pastille semi-conductrice ayant une orientation préférentielle de ses grains.

La compression réalisée dans le procédé de fabrication selon l'invention a lieu de préférence dès le début de l'étape de chauffage. Elle a lieu avec une charge ou force de compression de valeur donnée, pendant une durée donnée et sous une température donnée.

Après la fin de l'étape de compression axiale, l'étape de chauffage du frittage peut être immédiatement interrompue ou bien elle peut être poursuivie pendant une durée déterminée à une température maintenue ou une autre valeur de température déterminée, notamment selon la gradation de la poudre de matériau semi-conducteur et/ou l'épaisseur de la pastille que l'on désire produire.

De bons résultats ont aussi été obtenus en pratiquant d'abord une étape de compression axiale à froid, et en appliquant seulement après l'étape de chauffage du frittage proprement dit. Dans un tel mode de réalisation, il a été reconnu avantageux d'utiliser une technique de frittage en bogue, la pastille comprimée axialement étant placée sur une couche de poudre non compactée et recouverte enfin d'une couche de poudre non compactée.

L'étape de chauffage permet la croissance des grains au sein de la pastille. La présence du lit de poudre lors du frittage permet de limiter le contact entre la pastille et l'atmosphère lors du frittage. Les phénomènes de transport de matière en surface sont limités, par l'évaporation et la recondensation de matière de proche en proche en surface.

Le frittage en bogue permet d'obtenir un meilleur état de surface, c'est-à-dire une meilleure planéité en diminuant les aspérités des surfaces des faces opposées de la pastille. Le frittage en bogue permet également de diminuer la formation d'une phase parasite en surface qui pourrait se former si la pastille était directement en contact avec l'atmosphère du four lors du recuit.

Les faces du piston 4 et du fond de moule 4a présentent des états de surface permettant de respecter l'état de surface régulier et sans porosité ouverte qu'il a été possible d'obtenir avec la compression axiale d'une part et le chauffage lors du frittage proprement dit.

Il est ainsi parfaitement possible de déposer directement des électrodes sur les deux faces opposées "actives" de la pastille de matériau semi-conducteur et de déposer directement l'assemblage, des électrodes et de la pastille, sur un circuit intégré CMOS ainsi qu'il sera décrit plus loin.

Dans un mode particulier de réalisation, l'étape de compression axiale est précédée d'une étape lors de laquelle un dopant est incorporé au matériau semi-conducteur. Dans un mode préféré, le dopant est choisi parmi les composés iodure et est par exemple du Csl ou du BiI₃.

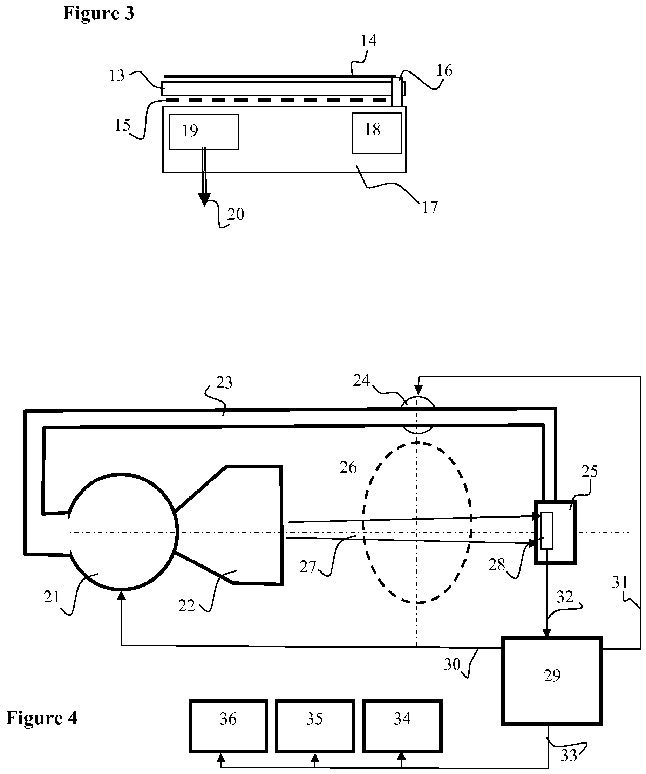

Sur la figure 3, on a représenté un détecteur à conversion directe de rayons X réalisé en utilisant une céramique ou pastille de matériau semi-conducteur 13 obtenue avec le procédé de fabrication de l'invention. La pastille de matériau semi-conducteur 13 présente deux faces parallèles et opposées, inférieure et supérieure en considérant la figure 3, qui sont d'un état de surface à la fois parfaitement plan et sans aspérités.

Il est alors possible de déposer par évaporation, ou au moyen de toute autre technique adaptée, une ou plusieurs couches métalliques, comme du Palladium, pour constituer respectivement une première électrode 14 sur la face supérieure et une seconde électrode 15 sur la face inférieure de la pastille de matériau semi-conducteur.

Dans un mode préféré de réalisation, la première électrode 14 est une électrode continue, plane et bidimensionnelle, qui est apte à être traversée par le flux de photons X incident. Selon l'énergie des photons X incidents, et donc de leur longueur d'onde, l'épaisseur axiale séparant la face supérieure de la face inférieure de la pastille 13 est déterminée pour maximiser l'absorption des photons X à travers la seconde électrode 15.

Cette seconde électrode 15 est réalisée non pas de façon continue bidimensionnelle, mais sous la forme de pavés délimitant au droit de chaque pavé un pixel de capteur d'image.

Selon les cas, la seconde électrode 15 est alors configurée de façon à ce que les pavés conducteurs soient arrangés :
- selon une direction linéaire ou autrement, mais unidimensionnelle, pour réaliser un capteur unidimensionnel d'image ;
- ou selon deux dimensions, sous divers arrangements choisis lors la conception du détecteur, pour réaliser un capteur bidimensionnel d'image 2D.

Chaque pavé conducteur de la seconde électrode est alors connecté par un réseau de lignes conductrices à un ensemble d'amplificateurs de signaux détectés pour chaque pixel. Ainsi qu'il est connu, l'arrangement 2D de pixels ainsi constitué peut être exploité directement ou par multiplexage notamment par un mécanisme d'adressage 2D en lignes et colonnes. A cette fin, le détecteur à conversion directe de l'invention comporte aussi un circuit intégré CMOS 17 sur la face supérieure duquel la pastille de matériau semi-conducteur 13, avec ses électrodes 14 et 15, est disposée et fixée.

Le circuit intégré CMOS 17 comporte principalement un circuit 18 de détection et un circuit 19 de traitement de signal. Le circuit 18 est connecté à chaque pixel de détection des charges électriques. Pour chaque pixel du détecteur, il comporte un préamplificateur et un circuit de mise en forme sensibles à la charge produite sur chaque pixel de la pastille 13. Le circuit 19 est doté de moyens pour produire des signaux électriques représentatifs de l'intensité des rayons X reçus dans les divers pixels de la pastille semi-conductrice.

Dans ce but, les électrodes 14 et 15 de la pastille de matériau semi-conducteur 13 sont connectées électriquement à des pavés conducteurs d'entrée (non représentés) du circuit intégré CMOS 17 par des liaisons électriques. Les pavés conducteurs d'entrée du circuit intégré CMOS 17 sont connectés électriquement aux circuits 18 de détection convenablement polarisé ainsi qu'il est connu.

On n'a pas décrit ici l'ensemble des circuits d'élaboration nécessaires du circuit intégré CMOS 17, mais seulement ceux nécessaires à la compréhension de l'invention. Les signaux électriques représentatifs de l'intensité des rayons X reçus produits par les circuits 18 sont disponibles sur des bornes de sortie 20 du circuit intégré complexe 13-20 ainsi constitué qui constitue un détecteur à conversion directe de rayons X.

Sur la figure 4, on a représenté schématiquement un appareil de radiologie dentaire qui utilise avantageusement au moins un détecteur à conversion directe de rayons X comme décrit en référence sur la figure 3.

Le capteur équipé de la pastille selon l'invention peut être utilisé dans un système d'imagerie dentaire extraoral. Une source 21 de rayons X est montée sur un bras 23 mobile sur un support 24 sur lequel il peut être entraîné en mouvement. En regard de la source 21 de rayons X, le bras 23 porte un équipement 25 destiné à produire au moins un signal d'image produit à l'aide d'au moins un capteur intégrant un détecteur à conversion directe 28 de rayons X constitué selon ce qui a été décrit plus haut.

Le capteur équipé de la pastille selon l'invention peut être utilisé dans un système d'imagerie dentaire intraoral. Le capteur est alors positionné dans la bouche du patient derrière la dent à radiographier. La source de rayons X est positionnée sur un bras ajustable. Elle est positionnée contre la joue du patient juste avant l'exposition.

Le détecteur à conversion directe 28 de rayons X est opposé à la source 21 de rayons X équipée d'un ensemble 22 assurant le filtrage et la collimation des rayons X incidents. Les rayons X 27 traversent alors une région d'analyse 26 comme la tête d'un patient, ou une partie de sa mâchoire, à examiner pour venir frapper le détecteur à conversion directe 28 de rayons X 27.

L'appareil de radiologie dentaire ainsi constitué comporte aussi un calculateur 29 pour réaliser notamment un circuit de commande 29, 31 pour exécuter au moins un tir de rayons X. Le calculateur 29 comporte principalement :
- des moyens pour produire des signaux de commande 30 de la source 21 de rayons X et de son ensemble de filtrage et de collimation 22 ;
- des moyens pour produire des signaux 31 de commande de l'entraînement du bras 23, si besoin, de façon à exécuter des balayages déterminés de la région d'analyse 26 en synchronisme avec des séquences de tirs de rayons X déterminés par la source 21 de rayons X ;
- des moyens pour recevoir et traiter les divers signaux électriques 32 représentatifs de l'intensité des rayons X reçus dans les divers pixels de la pastille semi-conductrice du détecteur à conversion directe de rayons X 28 ;
- des moyens pour en déduire par visualisation, impression et/ou enregistrement au moins une représentation graphique sur la base de la pluralité de signaux électriques produits par ledit au moins un détecteur à conversion directe de rayons X.

Ces derniers moyens du calculateur 29 sont ainsi connectés à un dispositif d'affichage graphique 34, à une imprimante graphique 35 et/ou à un système 36 de stockage et de consultation directe ou par le moyen d'un réseau de communication.

On note que ce genre d'appareil de radiologie fonctionne avec des récurrences de fréquence élevée qui exigent des performances que la pastille de matériau semi-conducteur obtenue par le procédé de fabrication de l'invention permet d'atteindre ainsi qu'on va le montrer plus avant.

### EXEMPLES

### Exemple 1

Pour des matériaux semi-conducteurs polycristallins, on a utilisé des paramètres suivants :
Température de frittage : 100°C.
Charge de compression axiale : 300 Mega (3.10⁸) Pascals.
Durée de mise en charge à cette température : 20 heures.

### Exemple 2

Pour des matériaux semi-conducteurs polycristallins, on a utilisé des paramètres suivants :
Température de frittage : comprise entre 70°C et 130°C.
Charge de compression axiale : comprise entre 100 Mega Pascals et 800 Mega Pascals.
Durée de mise en charge à cette température : au moins 1 heure.

### Exemple 3

Pour de l'iodure de mercure HgI₂, on a utilisé des paramètres suivants :
Température de frittage : inférieure à 200°C.
Charge de compression axiale : inférieure à 1000 Mega Pascals.
Durée de mise en charge à cette température : au moins 1 heure.

### Exemple 4

Pour de l'iodure de mercure HgI₂, on a utilisé des paramètres suivants :
Température de frittage : comprise entre 70°C et 200°C.
Charge de compression axiale: comprise entre 100 et 1000 Mega Pascals.
Durée de mise en charge à cette température : au moins 1 heure.

### Exemple 5

Pour des matériaux semi-conducteurs choisis parmi PbI₂, et Hgl2 on a ajouté un dopant à base d'un composé halogéné pris parmi notamment Csl, BiI₃, CdI₂, SnCl₂ et AgI. Pour des matériaux semi-conducteurs tels que PbO on a effectué un dopage par ajout d'un composé oxyde.

La concentration indiquée est de l'ordre de quelques pourcents.

### Essais des pastilles et détecteurs à conversion directe

*Effets de la pureté de la poudre de départ sur la sensibilité de la détection des rayons X.*

Dans une série d'essais effectués, la poudre utilisée pour réaliser les céramiques était une poudre commerciale vendue par la société "Sigma Aldrich" de pureté égale à 99,0% (référence 221090 ACS reagent, ≥99,0%).

A titre comparatif, des pastilles ont aussi été réalisées avec une poudre commerciale de pureté 99,999% (référence fournisseur 203785, 99,999% trace metal basis).

Le tableau 1 ci-dessous présente les mesures de courant d'obscurité et de sensibilité pour des pastilles réalisées avec de la poudre de puretés 99,0% et 99,999%. Il n'est pas possible pour des raisons physiques de connaître la dose exacte de rayons X reçue par l'échantillon. On ne connaît que la dose émise par la source. Cependant, les mesures de sensibilité effectuées pour deux échantillons de mêmes dimensions et de même épaisseur sont comparables. On a donc comparé la sensibilité relative d'un échantillon par rapport à l'autre.

**Tableau 1 : étude de la pureté**

| | Courant d'obscurité (nA/cm²) | Sensibilité relative |
|---|---|---|
| Pureté 99,0% | 251.6 (±25%) | 3,94 |
| Pureté 99,999% | 215.9 (±19%) | 1,0 |

Le courant d'obscurité doit être le plus faible possible afin d'avoir le meilleur rapport signal à bruit. La sensibilité traduit le nombre de charges collectées rapporté à la dose de rayons X et à la surface des électrodes. On cherche à maximiser cette grandeur.

Les pastilles réalisées à partir de la poudre à 99,0%, c'est-à-dire avec une pureté "médiocre" présentent un courant d'obscurité équivalent aux pastilles préparées avec une poudre plus pure, et surtout une plus grande sensibilité.

### Analyse cristallographique

Une analyse cristallographique a montré que le procédé selon l'invention a pour effet de faciliter la croissance des grains et la résorption de la porosité. Les grains ne sont pas seulement soudés les uns aux autres. Les plus petits grains sont en plus "absorbés" par les plus gros. Cette croissance des plus gros grains au détriment des plus petits grains se manifeste dans n'importe quelle opération de frittage, mais le procédé selon l'invention permet d'accroître son efficacité du fait de la charge appliquée.

En conséquence, le matériau polycristallin ainsi obtenu a une porosité beaucoup plus faible qu'un matériau obtenu par simple frittage sans mise sous charge ou par simple mise sous charge sans frittage.

Deuxièmement, lorsque les grains ont une structure cristalline anisotrope, le maintien d'une compression ou charge axiale pendant le frittage permet de favoriser la croissance des grains ayant une énergie mécanique minimale et il permet ainsi d'obtenir un matériau polycristallin dans lequel les grains ont une orientation préférentielle.

Dans le cas du HgI₂, cela permet d'obtenir un matériau avec une orientation telle que la direction de plus grande conductivité électrique est parallèle à l'axe de pression. La moindre porosité et l'orientation préférentielle des grains induite par le frittage sous charge permettent d'améliorer les propriétés de transport électrique par rapport à un frittage simple ou à un compactage sans frittage.

Troisièmement, la mise sous charge, en évitant l'évaporation, permet d'obtenir une meilleure planéité et une moindre rugosité des surfaces du matériau polycristallin, ce qui permet ultérieurement un meilleur contact entre des électrodes et les surfaces du matériau et de diminuer les défauts à la surface du matériau qui risqueraient de dégrader l'efficacité de collecte de charges électriques.

### Etude micro-structurale

On compare la microstructure de diverses pastilles de matériau semi-conducteur pour en déduire l'avantage de l'orientation par compression axiale. On compare de la poudre de HgI₂, une pastille frittée et une pastille frittée sous charge. L'orientation préférentielle des grains d'une pastille frittée sous charge et d'une poudre a été analysée par diffraction des rayons X.Pour comparer la proportion d'une structure orientée selon l'axe cristallographique C, on a mesuré l'aire sous les raies de diffraction caractéristiques de cet axe cristallographique (raies de type (00x), où x est un nombre entier positif non nul). Le tableau 2 ci-dessous présente les résultats pour une poudre de HgI₂, une pastille frittée de l'état de la technique et une pastille frittée sous charge selon l'invention. L'intensité relative est calculée comme le rapport de la raie de mesure (de type (00x)) à une raie de référence (raie (102)).

**Tableau 2 : intensité relative des raies caractéristiques de l'axe C**

| Intensité relative | Poudre | Frittée | Frittée sous charge |
|---|---|---|---|
| Raie (102) de référence | 1 | 1 | 1 |
| Raie (002) | 0.27 | 1.52 | 5.18 |
| Raie (004) | 0.22 | 1.23 | 3.13 |
| Raie (006) | 0.06 | 1.37 | 1.57 |

Dans le cas de la pastille ou céramique frittée sous charge réalisée selon l'invention, on obtient les valeurs les plus importantes pour les raies caractéristiques de l'axe cristallographique C, c'est-à-dire de type (00*x*) (figures 2a et 2b). Il y a donc une orientation préférentielle la plus importante selon cet axe.

### Temps de retour à l'équilibre après une impulsion de rayons X ("lag")

Ainsi qu'on l'a exposé lors de la description de la figure 4, une grandeur importante d'un détecteur en imagerie impulsionnelle, par exemple en imagerie 3D, est le temps de retour à l'équilibre ou « lag » c'est-à-dire le temps nécessaire pour que le courant produit par une impulsion de rayons X revienne à zéro après la fin de l'impulsion. Pour pouvoir réaliser des images rapides, ce "lag" doit être le plus court possible.

Le "lag" sur des pastilles de matériau semi-conducteur réalisées selon l'invention, ainsi que sur des références : monocristal de CdTe et monocristal de HgI₂ a été mesuré. Le tableau 3 ci-dessous présente les résultats.

**Tableau 3 : mesure de lag**

| | Pastilles HgI₂ | Monocristal CdTe | Monocristal HgI₂ |
|---|---|---|---|
| Lag | 1 ms | 66 ms | 15 ms |

Les pastilles HgI₂ réalisées par frittage sous charge axiale selon le procédé de fabrication selon l'invention présentent le "lag" le plus faible.

### Linéarité dans le temps

Un autre critère de performance est la linéarité dans le temps du détecteur à conversion directe de rayons X.

La charge électrique collectée par la pastille de matériau semi-conducteur doit être proportionnelle à la dose de rayons X reçue. La linéarité des pastilles a été mesurée en les soumettant à un train de rayons X, c'est-à-dire une succession d'expositions aux rayons X intercalées avec des temps de pause. On a mesuré la quantité de charges collectées par la pastille de matériau semi-conducteur, quantité cumulée au cours du temps, en exposant le détecteur à conversion directe de rayons X sous un train d'ondes de rayons X, composé d'une séquence d'impulsions. Les caractéristiques du train d'ondes de rayons X étaient les suivantes :
Durée d'une impulsion : 50 ms.
Durée d'obscurité suivante : 50 ms.

Le cumul CCC des charges collectées en fonction du temps respecte avec une forte précision une relation linéaire pendant l'intervalle de durée d'exposition [0.28s] du détecteur à conversion directe de rayons X :
CCC = a*t, avec a = 35000/28 en unité de charges collectées par unité de temps (en seconde).

La quantité de charges collectées cumulée CCC augmente à partir de la valeur nulle avant le début de l'exposition au train d'ondes de rayons X de façon linéaire en fonction du temps. C'est un avantage pour une utilisation en imagerie 3D, c'est-à-dire que tous les tirs de rayons X réalisés permettent de collecter le même nombre de charges.

### Réalisation d'un détecteur à conversion directe de rayons X

L'épaisseur de la pastille de matériau semi-conducteur produite selon le procédé de l'invention dépend de la granulométrie de la poudre initiale et de la valeur de la force de compression appliquée.

Dans un exemple de réalisation d'un détecteur à conversion directe de rayons X, on a atteint une densité très proche de celle du monocristal HgI₂ (98%). La quantité de poudre est déterminée par la relation : masse de poudre = section x épaisseur x densité (avec une masse volumique de 6,36 g/cm³).

Dans cet exemple de réalisation, il n'a pas été nécessaire de faire de préformage, le moule disposé dans le four (figure 1) étant utilisé lors du frittage sous charge pour donner la forme finale de la pastille fabriquée selon l'invention.

On note que plus le frittage est long, plus grande sera la densité et plus grande sera la taille des grains.

La pastille sortie du four était de forme cylindrique. Elle a été sciée et ébavuré pour former un parallélépipède rectangle de dimensions 15 cm x 15 cm x 500 µm (épaisseur). Le détecteur à conversion directe de rayons X a été réalisé en évaporant deux électrodes conductrices de 50 nm sur les deux faces antagonistes de la pastille dont l'état de surface était au préalable convenablement nettoyée et l'ensemble a été déposé sur un circuit intégré CMOS ainsi qu'il a été décrit à l'aide de la figure 3.

## Revendications

1. Procédé de fabrication d'une pastille semi-conductrice pour un détecteur à conversion directe de rayons X, le procédé comportant :
une étape de mise en charge sous pression (3, 4, 4a) d'une poudre d'un matériau polycristallin semi-conducteur à base de PbI₂, de HgI₂ et/ou de PbO; et
une étape de chauffage (5-9) pendant une durée déterminée, **caractérisé en ce que** :
le taux de pureté de la poudre du matériau polycristallin semi-conducteur est supérieur ou égal à 99% et inférieur ou égal à 99.8%.

2. Procédé selon la revendication 1, **caractérisé en ce que** l'étape de mise en charge consiste à appliquer (3) une force de compression axiale (4, 4a) de la poudre du matériau polycristallin semi-conducteur d'une valeur assurant une orientation axiale (C) des grains du matériau polycristallin semi-conducteur selon la direction d'application (C) de ladite force de compression axiale.

3. Procédé selon la revendication 2, **caractérisé en ce que** la valeur de ladite force de compression axiale est comprise entre 100 et 1.000 MPa, et **en ce que** la durée de mise sous charge est égale à au moins une heure.

4. Procédé selon la revendication 1 ou 3, **caractérisé en ce que** la température de chauffage est comprise entre 70°C et 200°C, et **en ce que** la durée de chauffage est égale à au moins une heure.

5. Procédé selon la revendication 1, **caractérisé en ce que** l'étape de mise en charge est mise en oeuvre dès le début de l'étape de chauffage.

6. Procédé selon la revendication 5, **caractérisé en ce que** l'étape de mise en charge est mise en oeuvre durant toute l'étape de chauffage.

7. Pastille semi-conductrice (13) pour un détecteur à conversion directe de rayons X, ladite pastille comportant :
une poudre d'un matériau polycristallin semi-conducteur à base de PbI₂, de HgI₂ et/ou de PbO ;
la pastille semi-conductrice (13) étant fabriquée par un procédé comportant une étape de mise en charge sous pression de la poudre du matériau polycristallin semi-conducteur et une étape de chauffage pendant une durée déterminée , la pastille étant **caractérisée en ce que**
le taux de pureté de la poudre du matériau polycristallin semi-conducteur est supérieur ou égal à 99% et inférieur ou égal à 99.8%.

8. Détecteur à conversion directe de rayons X, **caractérisé en ce qu'**il comporte une pastille semi-conductrice (13) selon la revendication 7.

9. Détecteur selon la revendication 8, **caractérisé en ce qu'**il est associé à un circuit intégré semi-conducteur, et **en ce qu'**il comporte une première électrode (14) continue en contact avec une face d'entrée de la pastille (13), et une seconde électrode (15) constituée par une pluralité de pavés conducteurs en contact avec la face opposée de la pastille (13), de façon à fournir un arrangement unidimensionnel ou bidimensionnel de pixels, lesdites première et seconde électrodes étant connectées électriquement (16) avec le circuit intégré semi-conducteur (17) associé sur une face duquel la pastille semi-conductrice (13) a été déposée, le circuit intégré semiconducteur (17) étant arrangé (18, 19) de sorte qu'il produise une pluralité de signaux électriques (20) représentatifs de l'intensité des rayons X reçus dans les divers pixels de ladite pastille semi-conductrice.

10. Appareil de radiologie, notamment de radiologie dentaire, utilisant au moins un détecteur à conversion directe de rayons X selon l'une des revendications 8 ou 9, ledit appareil comportant de plus au moins une source de rayons X (21, 22) commandée et un circuit de commande (29, 30) pour exécuter au moins un tir de rayons X en direction dudit au moins un détecteur à conversion directe (28) de rayons X et pour en déduire (33) par visualisation (34), impression (35) et/ou enregistrement (36) au moins une représentation graphique sur la base de la pluralité de signaux électriques produits par ledit au moins un détecteur à conversion directe de rayons X (28).

11. Appareil de radiologie selon la revendication 10, **caractérisé en ce que** ledit appareil est un appareil de radiologie dentaire intraoral ou extraoral.

## Patentansprüche

1. Verfahren zum Herstellen eines Halbleiterchips für einen Detektor mit direkter Umwandlung von Röntgenstrahlen, wobei das Verfahren umfasst:
einen Schritt der Druckbelastung (3, 4, 4a) eines Pulvers eines polykristallinen Halbleitermaterials auf der Basis von PbI₂, HgI₂ und/oder PbO; und
einen Schritt des Heizens (5-9) während einer bestimmten Dauer, **dadurch gekennzeichnet, dass**:
der Reinheitsgrad des Pulvers des polykristallinen Halbleitermaterials größer als oder gleich 99% und kleiner als oder gleich 99,8% ist.

2. Verfahren nach Anspruch 1, **dadurch gekennzeichnet, dass** der Schritt des Belastens daraus besteht, eine axiale Kompressionskraft (4, 4a) auf das Pulver des polykristallinen Halbleitermaterials mit einem Wert auszuüben (3), der eine axiale Orientierung (C) der Körner des polykristallinen Halbleitermaterials in der Richtung der Ausübung (C) der axialen Kompressionskraft sicherstellt.

3. Verfahren nach Anspruch 2, **dadurch gekennzeichnet, dass** der Wert der axialen Kompressionskraft zwischen 100 und 1000 MPa liegt, und dass die Dauer der Belastung mindestens eine Stunde beträgt.

4. Verfahren nach Anspruch 1 oder 3, **dadurch gekennzeichnet, dass** die Heiztemperatur zwischen 70°C und 200°C liegt und dass die Heizdauer mindestens eine Stunde beträgt.

5. Verfahren nach Anspruch 1, **dadurch gekennzeichnet, dass** der Schritt des Belastens gleich zu Beginn des Schritts des Heizens durchgeführt wird.

6. Verfahren nach Anspruch 5, **dadurch gekennzeichnet, dass** der Schritt des Belastens während des gesamten Schritts des Heizens durchgeführt wird.

7. Ein Halbleiterchip(13) für einen Detektor mit direkter Umwandlung von Röntgenstrahlen, wobei der Halbleiterchip umfasst:
ein Pulver eines polykristallinen Halbleitermaterials auf der Basis von PbI₂, HgI₂ und/oder PbO;
der Halbleiterchip (13) durch ein Verfahren hergestellt wird, das einen Schritt der Druckbelastung des Pulvers des polykristallinen Halbleitermaterials und einen Schritt des Heizens während einer bestimmten Dauer umfasst, wobei der Chip **dadurch gekennzeichnet ist, dass** der Reinheitsgrad des Pulvers des polykristallinen Halbleitermaterials größer als oder gleich 99% und kleiner als oder gleich 99,8% ist.

8. Ein Detektor mit direkter Umwandlung von Röntgenstrahlen, **dadurch gekennzeichnet, dass** er einen Halbleiterchip (13) nach Anspruch 7 umfasst.

9. Detektor nach Anspruch 8, **dadurch gekennzeichnet, dass** er mit einer integrierten Halbleiterschaltung assoziiert ist und dass er eine erste durchgehende Elektrode (14), die mit einer Eingangsseite des Chips (13) in Kontakt steht, und eine zweite Elektrode (15) umfasst, die aus einer Vielzahl von Leiterplättchen gebildet ist, die mit der gegenüberliegenden Seite des Chips (13) in Kontakt stehen, um eine ein- oder zweidimensionale Anordnung von Pixeln zu liefern, wobei die erste und die zweite Elektrode elektrisch (16) mit der assoziierten integrierten Halbleiterschaltung (17) verbunden sind, auf dessen einen Seite der Halbleiterchip (13) aufgebracht wurde, wobei die integrierte Halbleiterschaltung (17) so angeordnet ist (18, 19), dass sie eine Vielzahl von elektrischen Signalen (20) erzeugt, die für die Intensität der Röntgenstrahlen repräsentativ sind, die in den verschiedenen Pixeln des Halbleiterchips empfangen werden.

10. Röntgengerät, insbesondere Zahnröntgengerät, das mindestens einen Detektor mit direkter Umwandlung von Röntgenstrahlen nach einem der Ansprüche 8 oder 9 verwendet, wobei das Gerät ferner mindestens eine gesteuerte Röntgenstrahlenquelle (21, 22) und eine Steuerschaltung (29, 30) zum Ausführen von mindestens einem Röntgenstrahlbeschuss in Richtung des mindestens einen Detektor mit direkter Umwandlung von Röntgenstrahlen (28) und zum Ableiten (33) durch Visualisierung (34), Drucken (35) und/oder Aufzeichnen (36) von mindestens einer grafischen Darstellung davon basierend auf der Vielzahl von elektrischen Signalen, die von dem mindestens einen Detektor mit direkter Umwandlung von Röntgenstrahlen (28) erzeugt werden.

11. Röntgengerät nach Anspruch 10, **dadurch gekennzeichnet, dass** das Gerät ein intraorales oder extraorales Dentalröntgengerät ist.

## Claims

1. A method of manufacturing a semiconductor chip for a direct X-ray conversion detector, the method comprising:
a step of pressure charging (3, 4, 4a) a powder of a polycrystalline semiconductor material that is based on PbI₂, HgI₂ and/or PbO; and
a step of heating (5-9) during a certain period of time, **characterized in that**:
the purity level of the powder of the polycrystalline semiconductor material is greater than or equal to 99% and less than or equal to 99.8%.

2. The method according to claim 1, **characterized in that** the step of charging consists in applying (3) an axial compression force (4, 4a) to the powder of the polycrystalline semiconductor material of a value ensuring an axial orientation (C) of the grains of the polycrystalline semiconductor material according to the direction of application (C) of said axial compression force.

3. The method according to claim 2, **characterized in that** the value of said axial compression force is between 100 and 1000 MPa, and **in that** the duration of the charging is equal to at least one hour.

4. The method according to claim 1 or 3, **characterized in that** the heating temperature is between 70°C and 200°C, and that the duration of the heating is at least one hour.

5. The method according to claim 1, **characterized in that** the step of charging is carried out at the beginning of the step of heating.

6. The method according to claim 5, **characterized in that** the step of charging is carried out during the entire step of heating.

7. A semiconductor chip (13) for a direct X-ray conversion detector, the semiconductor chip comprising:
a powder of a polycrystalline semiconductor material that is based on PbI₂, HgI₂ and/or PbO;
the semiconductor chip (13) being manufactured by a process comprising a step of pressure charging the powder of the polycrystalline semiconductor material and a step of heating during a certain period of time, the chip being **characterized in that** the purity level of the powder of the polycrystalline semiconductor material is greater than or equal to 99% and less than or equal to 99.8%.

8. A direct conversion X-ray detector, **characterized in that** it comprises a semiconductor chip (13) as claimed in claim 7.

9. The detector according to claim 8, **characterized in that** it is associated with a semiconductor integrated circuit, and **in that** it comprises a first continuous electrode (14) in contact with an input face of the chip (13), and a second electrode (15) constituted by a plurality of conductive pads in contact with the opposite face of the chip (13), so as to provide a one-dimensional or two-dimensional arrangement of pixels, said first and second electrodes being electrically connected (16) with the associated semiconductor integrated circuit (17) on one face on which the semiconductor wafer (13) has been deposited, the semiconductor integrated circuit (17) being arranged (18, 19) so that it produces a plurality of electrical signals (20) representative of the intensity of the X-rays received in the various pixels of said semiconductor wafer.

10. An X-ray apparatus, in particular a dental X-ray apparatus, using at least one direct conversion X-ray detector according to one of claims 8 or 9, said apparatus further comprising at least one controlled X-ray source (21, 22) and a control circuit (29, 30) for carrying out at least one X-ray shot in the direction of the at least one direct conversion X-ray detector (28) and for deriving (33) by displaying (34), printing (35) and/or recording (36) at least one graphical representation on the basis of the plurality of electrical signals produced by the at least one direct conversion X-ray detector (28).

11. The X-ray apparatus of claim 10, **characterized in that** the apparatus is an intraoral or extraoral dental X-ray apparatus.
